# EUROPÄISCHE PATENTSCHRIFT

(11) **EP 1 474 134 B1**
(45) Veröffentlichungstag und Bekanntmachung des Hinweises auf die Patenterteilung: **17.08.2011**
(21) Anmeldenummer: 03737262.0
(22) Anmeldetag: 22.01.2003
(51) Int. Cl.: A61K 31/403, A61K 31/155, A61P 9/04

(54) **KOMBINATIONSPRÄPARAT DES NATRIUM-WASSERSTOFF-AUSTAUSCH-INHIBITORS CARIPORIDE MIT RAMIPRIL ZUR VERHINDERUNG BZW ZUR HEMMUNG DER HERZINSUFFIZIENZ**
COMBINATION PREPARATION OF THE SODIUM-HYDROGEN EXCHANGE INHIBITOR CARIPORIDE WITH RAMIPRIL FOR PREVENTING OR BLOCKING HEART FAILURE
PREPARATION COMBINEE DE L'INHIBITEUR D'ECHANGE SODIUM-HYDROGENE CARIPORIDE ET DE RAMIPRIL POUR PREVENIR OU INHIBER L'INSUFFISANCE CARDIAQUE

(30) Priorität: 04.02.2002 DE 10204571
(43) Veröffentlichungstag der Anmeldung: 10.11.2004
(73) Patentinhaber: Sanofi-Aventis Deutschland GmbH, 65929 Frankfurt am Main (DE)
(72) Erfinder: LINZ, Wolfgang, 55129 Mainz (DE); SCHINDLER, Ursula, 65812 Bad Soden (DE)
(74) Vertreter: Michalski Hüttermann & Partner Patentanwälte
(86) Internationale Anmeldenummer: PCT/EP2003/000587
(87) Internationale Veröffentlichungsnummer: WO 2003/066045

(56) Entgegenhaltungen:
- EP-A- 0 855 392
- EP-A- 0 937 464
- DE-A- 19 734 693
- DE-A- 19 859 727
- RUETTEN HARTMUT ET AL: "The NHE1 inhibitor cariporide improves left ventricular morphology and function in rats with chronic heart failure in combination with the ACE inhibitor ramipril." CIRCULATION, [Online] Bd. 106, Nr. 19 Supplement, 5. November 2002 (2002-11-05), Seiten II-292, XP001147832 Abstracts from Scientific Sessions;Chicago, IL, USA; November 17-20, 2002 ISSN: 0009-7322 Gefunden im Internet: <URL:http://aha.agora.com/abstractviewer/a v_view.asp> [gefunden am 2003-04-29]
- LINZ WOLFGANG ET AL: "Long-term ACE inhibition doubles lifespan of hypertensive rats." CIRCULATION, [Online] Bd. 96, Nr. 9, 4. November 1997 (1997-11-04), Seiten 3164-3172, XP002239742 ISSN: 0009-7322 Gefunden im Internet: <URL:http://circ.ahajournals.org/cgi/conte nt/full/96/9/3164> [gefunden am 2003-04-28]

## Beschreibung

Die Erfindung beschreibt eine Kombination von Cariporide (HOE 642), einem Inhibitor des zellulären Natrium-Wasserstoff-Austauschers, mit dem ACE-Hemmer Ramipril, und ihre Anwendung in der Human- und Veterinärmedizin für die Verhinderung der Herzinsuffizienz.

In der WO 00 / 38 661 wird die Verwendung von Hemmern des Natrium-Wasserstoff-Austauschers (NHE) zur Herstellung eines Medikaments zur Verhinderung von altersbedingter Organ-Dysfunktion, altersbedingten Erkrankungen und zur Lebensverlängerung beschrieben. Dabei werden Inhibitoren des zellulären Natrium-Wasserstoff-Austauschers zur Herstellung eines Medikaments für die Verhinderung altersbedingter Funktionsstörungen und dysfunktioneller Veränderungen von Organen des Körpers und für die Verhinderung altersbedingter Erkrankungen und für die Lebensverlängerung unter Wahrung einer verbesserten Lebensqualität beschrieben. Als typischer Vertreter der NHE-Inhibitoren werden Cariporide und dessen pharmazeutisch verträgliche Salze benannt, zum Beispiel das Cariporide Mesilat mit der nachfolgenden Formel:

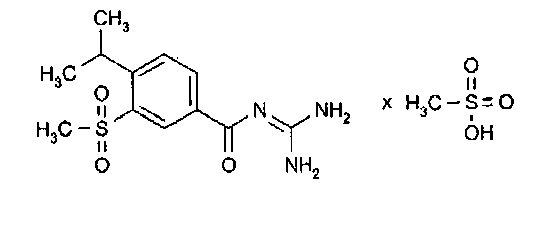

lm folgenden werden die freie Base des Cariporids und deren pharmazeutisch verträgliche Salze, einschließlich des Cariporide Mesilats, unter dem Begriff Cariporide zusammengefasst.

In der WO 00 / 38 661 wurden die biologischen Befunde an normotensiven gesunden Ratten erhoben. Im Gegensatz zu NHE-Inhibitoren sind Hemmer des Angiotensin Converting Enzymes (ACE) am Beispiel von Ramipril ohne jegliche protektive Wirkung auf die geschilderten krankhaften altersbedingten Organveränderungen in alten normotensiven Ratten. Weiterhin wird in der WO 00 / 38 661 die Kombination von NHE-Inhibitoren mit blutdrucksenkenden Medikamenten, wie mit ACE-Hemmem, Angiotensinrezeptorantagonisten usw. in Erwägung gezogen, um zusätzlich zur alterungsprotektiven Wirkung der NHE-Inhibitoren den möglichen Benefit einer blutdrucksenkenden Wirkung von Hypotensiva zu nutzen. Eine Steigerung einer lebensverlängemden Wirkung bzw, einer protektiven Wirkung auf pathologische altersbedingte Organveränderungen wurde nicht erwartet.

Aus der Literatur ist seit längerem bekannt, dass Ramipril mit der Formel 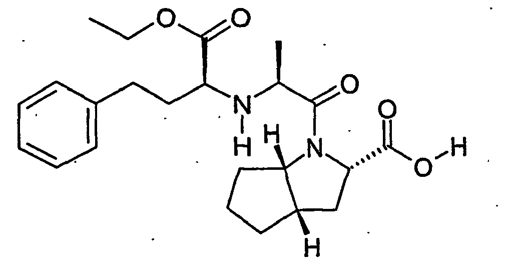 die infolge ihrer Hochdruckkrankheit verkürzte Lebensspanne von spontan hypertensiven Ratten signifikant verlängert.

In den vorliegenden Experimentalbefunden wird nun gezeigt, dass NHE-Inhibitoren allein und in Kombination mit ACE-Inhibitoren ebenfalls an spontan-hypertensiven Ratten (SHR) eine signifikante lebensverlängernde Wirkung und eine protektive Wirkung auf pathologische altersbedingte Organveränderungen verursachen. Um eine Wirkung der Blutdrucksenkung durch den ACE-Inhibitor Ramipril als sekundären Effekt für eine mögliche Lebensverlängerung auszuschließen, wurde Ramipril in unterschwelliger Dosis bezüglich Blutdrucksenkung angewandt. Dabei kam es in überraschender Weise in der Kombinationsbehandlung des NHE-Inhibitors Cariporide mit dem ACE-Inhibitor Ramipril bei nicht-blutdrucksenkender Wirkung zu signifikant günstigeren altersprotektiven Effekten. Die Kombinationsbehandlung ist beiden Einzelpräparaten signifikant überlegen.

Eine unterschwellige, nicht-blutdrucksenkende Dosis Ramipril liegt bei der Ratte üblicherweise in einem Bereich von 1 bis 50 µg pro kg Körpergewicht und pro Tag. Bei Menschen liegt eine unterschwellige Dosis Ramipril bei Verabreichung an eine Person von etwa 70 kg Körpergewicht im allgemeinen in einem Bereich von 0,1 bis 1,0 mg pro Tag und Mensch, bevorzugt 0,3 bis 0,7 mg pro Tag und Mensch, zum Beispiel bei 0,625 mg pro Tag und Mensch.

Für die Experimente wurden alte spontan hypertensive Ratten (Alter =18 Monate) entweder mit dem NHE-Inhibitor Cariporide, oder mit einer unterschwelligen, nicht blutdrucksenkenden Dosis des ACE-Inhibitors Ramipril oder mit einer entsprechenden Kombination beider Wirkstoffe behandelt. Das Modell dieser hochdruckkranken Ratten wird allgemein als repräsentativ für die menschliche essentielle Hypertonie angesehen. Für jede Versuchsgruppe wurden 27 hypertensive Ratten im Alter von 18 . Monaten eingesetzt. 3 Monate nach Behandlungsbeginn, als in der Placebo-Kontrollgruppe nur noch n = 7 überlebende Tiere vorhanden waren, wurde eine Interimsanalyse mit n = 7 Tieren aus jeder Gruppe durchgeführt. Die Herzen der mit niedrigen Dosen an Ramipril behandelten SHRs zeigten eine signifikante Verminderung der Herzkraft im Vergleich zu den Placebo-Kontrollherzen. Im Gegensatz hierzu verbesserte Cariporide und die Ramipril/Cariporide-Kombination die Herz-Funktion signifikant.

Ebenfalls wurde die mit der Herzinsuffizienz korrelierende myocardiale Fibrose signifikant durch Cariporide-Monotherapie sowie durch die Ramipril/Cariporide-Kombination vermindert. Die signifikant geringeren Herzgewichte zeigen ebenfalls die antifibrotische, gegen die Entstehung der Herzinsuffizienz gerichtete Wirkung der Ramipril/Cariporide-Kombination und von Cariporide.

Die Einzelpräparate Ramipril und Cariporide führen zu einer Verbesserung der Überlebensrate, im Vergleich zur Placebo-Behandlung. Signifikante Unterschiede in den Überlebensraten von Cariporide und Ramipril konnten dabei nicht nachgewiesen werden. Überraschenderweise führt die Cariporide-Ramipril-Kombination zu einer signifikanten Erhöhung der Überlebensrate im Vergleich mit den durch die Einzelverbindungen erzeugten Effekten.

Cariporide kann somit erfindungsgemäß zusammen mit Ramipril am Tier, bevorzugt am Säugetier, und insbesondere am Menschen in vorteilhafter Weise als Arzneimittel verwendet werden. Gegenstand der Erfindung ist die Kombination von Cariporide und Ramipril zur Anwendung in der Humanmedizin und Veterinärmedizin, zum Beispiel beim Hund. Die Kombination der beiden Wirkstoffe kann derart erfolgen, dass Cariporide und Ramipril zusammen in einem Medikament (Kombinationspräparat) verabreicht werden

Das Gewichtsverhältnis von Cariporide zu Ramipril in den erfindungsgemäßen Kombinationen liegt üblicherweise in einem Bereich von 1 : 0,0001 bis 1 : 1, bevorzugt zwischen 1 : 0,0001 und 1 : 0,1, zum Beispiel zwischen 1 : 0,0005 und 1 : 0,01.

Weiterhin sind Gegenstand der vorliegenden Erfindung pharmazeutische Zubereitungen, die als aktiven Bestandteil sowohl Cariporide als auch Ramipril neben üblichen, pharmazeutisch einwandfreien Träger- und Hilfsstoffen enthalten. Die pharmazeutischen Zubereitungen enthalten normalerweise 0,01 bis 90 Gewichtsprozent Cariporide und Ramipril. Die Herstellung der pharmazeutischen Zubereitungen kann in an sich bekannter Weise erfolgen. Dazu werden die Wirkstoffe zusammen mit einem oder mehreren festen oder flüssigen galenischen Trägerstoffen und/oder Hilfsstoffen in eine geeignete Darreichungsform bzw. Dosierungsform gebracht, die dann als Arzneimittel in der Humanmedizin oder Veterinärmedizin verwendet werden kann. Entsprechendes gilt auch für erfindungsgemäß eingesetzte pharmazeutische Zubereitungen, die die Wirkstoffe Cariporide und Ramipril separat enthalten.

Beansprucht werden insbesondere Kombinationspräparate aus Cariporide und Ramipril zur Verhinderung bzw. Hemmung des weiteren Fortschreitens der Herzinsuffizienz bzw. zur partiellen Regression der Erkrankung.

Gegenstand der vorliegenden Erfindung ist auch die Verwendung von Cariporide und Ramipril zur Herstellung einer pharmazeutischen Zubereitung und deren Verwendung als Medikament zur Verhinderung bzw. Hemmung des weiteren Fortschreitens der Herzinsuffizienz bzw. zur partiellen Regression der Erkrankung.

Die Dosierung der zu verabreichenden Wirkstoffe Cariporide und/oder Ramipril hängt vom Einzelfall ab und ist wie üblich für eine optimale Wirkung den Gegebenheiten des Einzelfalls anzupassen. So hängt sie natürlich ab von der Häufigkeit der Verabreichung und von der Art der jeweils zur Therapie oder Prophylaxe eingesetzten galenischen Formulierungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder chronisch therapiert wird oder Prophylaxe betrieben wird. Wenn die Erfindungen am Tier, bevorzugt am Säugetier, und insbesondere am Menschen als Arzneimittel verwendet werden, so kann die Dosierung des Ramiprils im Bereich von 0,1 bis 10 mg pro Tag und pro Mensch (bei etwa 70 kg Körpergewicht) variieren, bevorzugt von 0,1 bis 5 mg pro Tag und Mensch, besonders bevorzugt von 0,2 bis 2,5 mg pro Tag und Mensch. Die Ramipril-Dosis kann auch in einem unterschwelligen Bereich von 0,1 bis 1 mg pro Tag und Mensch gegeben werden, bevorzugt von 0,3 bis 0,7 mg pro Tag und Mensch. Bei Cariporide kann die Dosis zwischen 50 mg und 1 g pro Tag und Mensch variieren, bevorzugt zwischen 100 bis 500 mg pro Tag und Mensch. Beispielsweise kann die Ramipril-Dosis 0,625 mg pro Tag und Mensch betragen und die Cariporide-Dosis 200 mg pro Tag und Mensch.

Bei der erfindungsgemäßen Kombinationsbehandlung können Cariporide und Ramipril in geringeren Dosen verabreicht werden als bei Verabreichung nur eines der beiden Wirkstoffe.

Bei der erfindungsgemäßen Kombinationsbehandlung kann die Tagesdosis der Wirkstoffe auf einmal verabreicht werden oder sie kann auf mehrere, zum Beispiel zwei, drei oder vier, Verabreichungen aufgeteilt werden.

Arzneimittel, die erfindungsgemäße Kombinationen von Cariporide und Ramipril enthalten, können oral, parenteral, z. B intravenös, rektal, transdermal oder topisch appliziert werden, wobei die bevorzugte Applikation vom Einzelfall abhängig ist.

Welche Hilfsstoffe für die gewünschte Arzneimittelformulierung geeignet sind, ist dem Fachmann auf Grund seines Fachwissens geläufig. Neben Lösemitteln, Gelbildnem, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wässrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

### Versuchsdaten

Untersucht wurde der Effekt einer Kombination aus Ramipril und Cariporide auf die Überlebensrate, Fibrose und die Herzgewichte von Ratten mit genetisch bedingter Hypertonie.

Hierfür wurden bei dem Experiment alte spontan hypertensive Ratten (Alter = 18 Monate) entweder mit dem NHE-Inhibitor Cariporide, oder mit einer unterschwelligen, nicht blutdrucksenkenden Dosis des ACE-Inhibitors Ramipril oder mit einer entsprechenden Kombination beider Wirkstoffe behandelt. Für jede der vier Behandlungsmethoden wurden 27 hypertensive Ratten im Alter von 18 Monaten eingesetzt. Als tägliche Dosis wurden 400 mg/ kg Körpergewicht Cariporide über das Futter (0,6% Cariporide in Standardrattendiät) und 10 µg/kg Körpergewicht Ramipril über das Trinkwasser verabreicht. 3 Monate nach Behandlungsbeginn, als in der Placebo-Kontrollgruppe nur noch n = 7 übergebende Tiere vorhanden waren, wurde eine Interimsanalyse mit n = 7 Tieren aus jeder Gruppe durchgeführt. Bei den restlichen, noch lebenden Ratten pro Gruppe wurden durchgehend ab dem 18. Monat die oben angegebenen Behandlungsmethoden angewandt. Zum Nachweis, dass die Dosis des Ramiprils nicht blutdrucksenkend war, wurde regelmäßig der Blutdruck von den Ratten nach der Tail-Cuff-Methode gemessen, wobei in den 4 Behandlungsgruppen keine signifikanten Unterschiede festzustellen waren.

Bei der Untersuchung der Überlebensrate wurden für jede Versuchsgruppe also 20 Ratten im Alter von 18 Monaten eingesetzt (Tabelle 1). Die Beobachtungsergebnisse sind in der Tabelle 2 zusammengefasst.

**Tab. 1: Versuchsgruppen**

| **Gruppe** | **Behandlung** | **N** |
|---|---|---|
| 1 | Placebo | 20 |
| 2 | Ramipril | 20 |
| 3 | Cariporide | 20 |
| 4 | Kombination | 20 |

**Tab. 2: Anzahl überlebender Ratten**

| **Alter (Monat)** | **Placebo** | **Cariporide** | **Ramipril** | **kombi** |
|---|---|---|---|---|
| 18 | 20 | 20 | 20 | 20 |
| 19 | 10 | 18 | 18 | 20 |
| 20 | 4 | 16 | 15 | 18 |
| 21 | 0 | 12 | 11 | 18 |
| 22 | 0 | 8 | 9 | 18 |
| 23 | 0 | 7 | 4 | 16 |
| 24 | 0 | 7 | 0 | 14 |
| 25 | 0 | 5 | 0 | 13 |
| 26 | 0 | 0 | 0 | 8 |
| 27 | 0 | 0 | 0 | 7 |
| 28 | 0 | 0 | 0 | 3 |
| 29 | 0 | 0 | 0 | 0 |

Im folgenden wird untersucht, ob für die Präparatgruppen eine Verbesserung der Überlebensrate gegenüber Placebo sowie für die Kombination eine Verbesserung der Überlebensrate gegenüber den Einzelpräparaten nachweisbar ist.

### Deskriptive Analyse

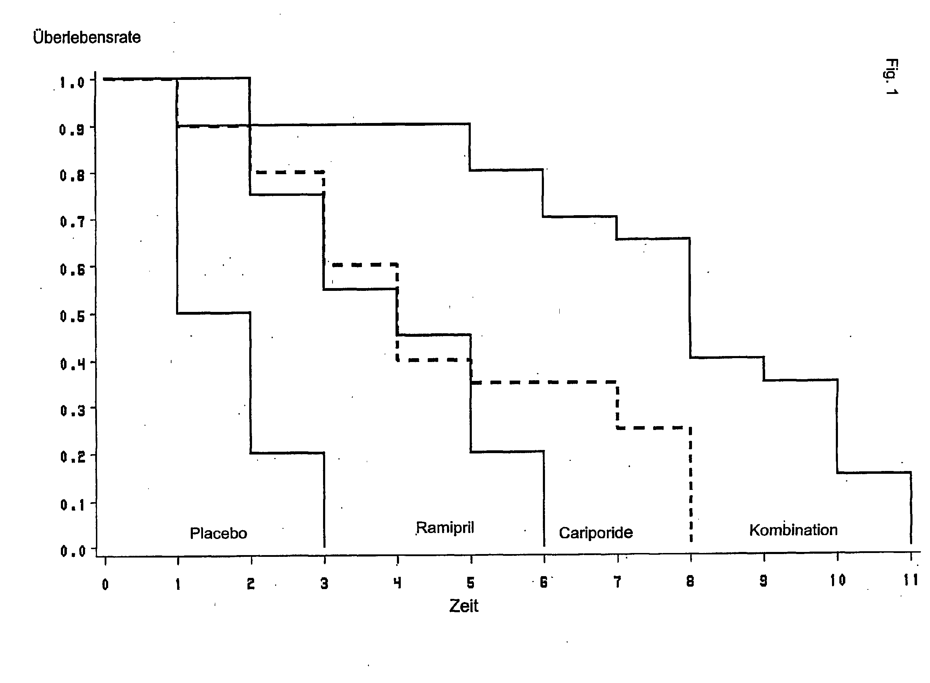

Fig. 1 zeigt den Verlauf der Überlebensrate für alle Behandlungsgruppen im Detail. Deutliche Unterschiede zwischen Placebo und Präparatgruppen einerseits, sowie zwischen den Einzelpräparaten und der Kombination sind bereits nach einer Versuchsdauer von 3 - 4 Monaten erkennbar.

Die Kombinationsbehandlung lieferte über den gesamten Versuchszeitraum hinweg die besten Ergebnisse. Ebenso ist ersichtlich, dass die Placebokontrolle allen Behandlungen deutlich unterlegen war.

### Statistische Analyse

Die Tabelle 3 zeigt die Ergebnisse aller paarweisen Vergleiche von jeweils zwei Behandlungsgruppen. Die Testergebnisse gelten auf einem multiplen Signifikanzniveau von 5%.

**Tab. 3: Holm-adjustierte Log Rank Tests.**

| **Gruppe** | **vs. Gruppe** | **Test** |
|---|---|---|
| Placebo | Cariporide | * |
| | Ramipril | * |
| | Kombi | * |
| Kombi | Ramipril | * |
| | Cariporide | * |
| Ramipril | Cariporide | n.s. |

| | | |
|---|---|---|
| * bedeutet signifikanter Unterschied, n.s. = nicht signifikant | | |

Alle Präparatgruppen sind statistisch signifikant von der Placebokontrolle verschieden, was zusammen mit Fig. 1 als Effektivität der Behandlungen gewertet werden kann. Fig. 1 lässt zudem deutliche Vorteile der Kombination im Vergleich zu den Einzelpräparaten erkennen.

### Mediane Überlebenszeit

Die Tabelle 4 zeigt Schätzungen für die mediane Ausfallzeit, dem Zeitpunkt, an dem die Überlebensrate in der Versuchstierpopulation gerade 50% erreicht.

**Tab. 4: Mediane Überlebenszeit**

| **Gruppe** | **Median** | **95%-Intervall** |
|---|---|---|
| Placebo | 1.5 | [1, 2) |
| Cariporide | 4.0 | [3,7) |
| Ramipril | 4.0 | [3, 5) |
| kombi | 8.0 | [6, 10) |

Demnach ist der Anteil überlebender Tiere in der Placebo-Gruppe bereits nach 1.5 Monaten auf 50 % gesunken, während dies bei einer Kombinationsbehandlung erst nach 8 Monaten zu erwarten ist.

Die Einzelpräparate erreichen mit einer medianen Überlebenszeit von 4 Monaten weniger als die Hälfte des Kombinationspräparates.

### Statistische Methode

Die statistische Analyse wurde im wesentlichen mit der SAS/STAT Prozedur LlFETEST [SAS Institute Inc (1989): SAS/STAT User's Guide, Version 6, 4th Edition, Volume 2; Cary, NC; SAS Institute Inc.] durchgeführt. Die Log Rank Tests wurden paarweise für jeweils zwei Vergleichsgruppen ausgeführt und zur Einhaltung eines multiplen Signifikanzniveaus von 5 % nach S. Holm (1979): A Simple Sequentally Rejective Multiple Test Procedure; Scand. J. Statist.; 6, pp. 65-70 adjustiert.

### Schlussfolgerung für die Überlebensraten

Die Einzelpräparate Ramipril und Cariporide führen zu einer statistisch signifikanten Verbesserung der Überlebensrate, im Vergleich zur Placebo Behandlung. Unterschiede in den Überlebensraten von Cariporide und Ramipril konnten auf dem gewählten Signifikanzniveau nicht nachgewiesen werden. Die Kombinationsbehandlung ist beiden Einzelpräparaten signifikant überlegen.

Durch den chronisch vorliegenden Hypertonus kommt es bei den alten spontan hypertensiven Tieren zu Umbauprozessen im Herzmuskelgewebe, die im wesentlichen denen entsprechen wie sie beim Menschen ablaufen (In einer frühen Phase (10. - 15. Lebensmonat) kompensierte Herzinsuffizienz mit Hypertrophie (NYHA-Stadium I-II), in der späteren Phase (16. - 21. Lebensmonat) dekompensierte Herzinsuffizienz mit Kammerdilatation und Fibrose (NYHA-Stadium III-IV)).

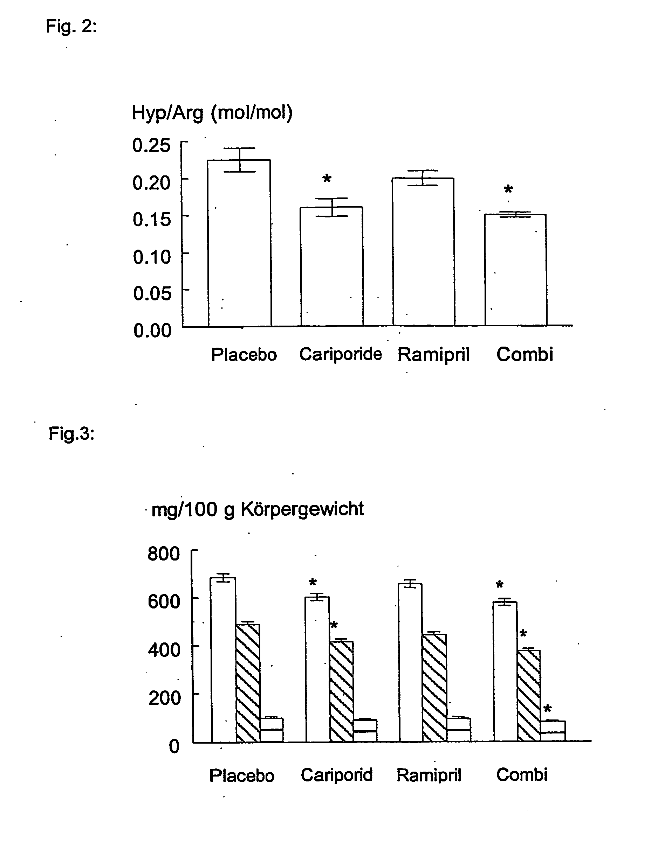

Wie oben bereits erwähnt wurde 3 Monate nach Behandlungsbeginn, als in der Placebo-Kontrollgruppe nur noch n = 7 überlebende Tiere vorhanden waren, eine Interimsanalyse mit n = 7 Tieren aus jeder Gruppe durchgeführt. Bestimmt wurde unter - anderem das Gesamtherzgewicht und die Gewichte des linken und rechten Ventrikels und das molare Verhältnis von Hydroxyprolin zu Ariginin im Herzgwebe (bestimmt durch HPLC), das ein Marker für die myokardiale Fibrosierung ist. Interessanterweise konnte eine schon bestehende Fibrose durch die spät-einsetzende Behandlung mit Cariporide aber nicht mit Ramipril vermindert werden, eine noch stärkere Verminderung der Fibrose konnte bei der Ramipril/Cariporide-Kombination beobachtet werden (Fig. 2). Diese Effekte schlagen sich auch in den Herzgewichten nieder (Fig. 3).

In den Zeichnungen wurden folgende Beschriftungen und Kennzeichnungen vorgenommen:
Fig.1: Kaplan-Meier-Überlebenszeiten
   Y-Achse: Überlebensrate (1 = 100%)
   X-Achse: Zeit in Monaten ab Behandlungsbeginn (Behandlungsbeginn = 0, identisch mit dem 18. Lebensmonat)
Fig.2: Einfluss einer späten Behandlung mit Cariporide und Ramipril auf die myokardiale Fibrose von alten spontan hypertensiven Ratten
   Y-Achse: Präparatgruppen
   X-Achse: Hyp/Arg ist das Verhältnis von Hydroxyprolin zu Arginin und gilt als Marker für die Fibrosierung
   * bedeutet p < 0,05 vs Placebo
   n = 7 pro Gruppe
Fig.3: Einfluss einer späten Behandlung mit Cariporide und Ramipril auf das Herzgewicht von spontan hypertensiven Ratten
   Y-Achse: Präparatgruppen
   X-Achse: mg Herzgewicht pro 100g Körpergewicht
   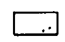 Gesamtherz
   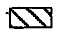 Linker Ventrikel
   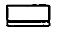 Rechter Ventrikel
   * p < 0,05 vs Placebo
   n = 7 pro Gruppe

### Zusammenfassung der Ergebnisse

Durch die vorliegenden Ergebnisse wird gezeigt, dass die Therapie mit einer Kombination aus dem NHE1-Inhibitor Cariporide und Ramipril lebensverlängemd wirkt und altersbedingte Organschädigungen, insbesondere der Herzinsuffizienz in ihrem Fortschreiten hemmt, bzw. wieder partiell rückgängig macht, auch bei späteinsetzender Therapie und/oder Bluthochdruckerkrankungen. -

## Patentansprüche

1. Kombinationspräparat aus Cariporide und Ramipril.

2. Kombinationspräparat aus Cariporide und Ramipril nach Anspruch 1 zur Verhinderung bzw. Hemmung des weiteren Fortschreitens der Herzinsuffizienz bzw. zur partiellen Regression der Erkrankung in der Human- oder Veterinärmedizin.

3. Verwendung von Cariporide in Kombination mit Ramipril zur Herstellung eines Medikaments zur Verhinderung bzw. Hemmung des weiteren Fortschreitens der Herzinsuffizienz bzw. zur partiellen Regression der Erkrankung in der Human- oder Veterinärmedizin.

## Claims

1. A cariporide and ramipril combination product.

2. A cariporide and ramipril combination product as claimed in claim 1 for preventing or inhibiting further progression of heart failure and for partial regression of the disorder in human or veterinary medicine.

3. The use of cariporide in combination with ramipril for producing a medicament for preventing or inhibiting further progression of heart failure and for partial regression of the disorder in human or veterinary medicine.

## Revendications

1. Préparation en combinaison de cariporide et de ramipril.

2. Préparation en combinaison de cariporide et de ramipril selon la revendication 1, pour empêcher ou inhiber la poursuite de la progression de l'insuffisance cardiaque, ou pour la régression partielle de la maladie en médecine humaine ou vétérinaire.

3. Utilisation de cariporide en combinaison avec du ramipril pour la fabrication d'un médicament destiné à empêcher ou inhiber la poursuite de la progression de l'insuffisance cardiaque, ou pour la régression partielle de la maladie en médecine humaine ou vétérinaire.
